# EUROPEAN PATENT APPLICATION

(11) **EP 4 663 772 A1**
(43) Date of publication of application: **17.12.2025**
(21) Application number: 24194795.1
(22) Date of filing: 15.08.2024
(51) Int. Cl.: C12Q 1/686, C12Q 1/70, C12Q 1/6806

(54) **METHOD OF DETECTING A VIRUS PATHOGEN IN A DAIRY PRODUCT**

(30) Priority: 12.06.2024 EP 24181749
(71) Applicant: QIAGEN GmbH, 40724 Hilden (DE)
(72) Inventor: DI PASQUALE, Francesca, 40724 Hilden (DE); DONOHOE, Colin, 40724 Hilden (DE); LAUREYS, Nina Sophie, 40724 Hilden (DE); PEIST, Ralf, 40724 Hilden (DE)
(74) Representative: Witte, Weller & Partner Patentanwälte mbB

(57) **Abstract**

The present invention relates to a digital PCR method of detecting a virus pathogen in a diary product or water, and a use associated therewith.

## Description

The present invention relates to a method of detecting a virus pathogen in a diary product or water, a kit for carrying out said method, and a use associated therewith.

### FIELD OF THE INVENTION

The present invention relates to the field of molecular biology, more particularly to the detection of virus pathogens via nucleic acid amplification.

### BACKGROUND OF THE INVENTION

It has long been known that pathogens that have their original natural host in the animal kingdom can be transmitted to humans under certain circumstances. This process, known as 'zoonosis', can be observed for a wide variety of pathogens, such as, for example, bacteria, viruses, parasites, or prions. Major modern diseases such as Ebola and salmonellosis are zoonoses. HIV was a zoonotic disease transmitted to humans in the early part of the 20th century, though it has now evolved into a separate human-only disease. Human infection with animal influenza viruses is rare, as they do not transmit easily to or among humans. However, avian and swine influenza viruses in particular possess high zoonotic potential, and these occasionally recombine with human strains of the flu and can cause pandemics such as the 2009 swine flu.

Influenza A virus subtype H5N1 (A/H5N1) is a subtype of the influenza A virus, which causes influenza, predominantly in birds. It is enzootic (maintained in the population) in many bird populations, and also panzootic (affecting animals of many species over a wide area). A/H5N1 virus can also infect mammals, including humans, that have been exposed to infected birds; in these cases, symptoms are frequently severe or fatal.

In 2024 a moderate growth in infections in cattle through the variant H5N1 has been observed. In late March 2024, the H5N1 virus was found in dairy herds in Texas and Kansas. This is the second time the virus has been detected in a human in the United States and the first time it appears to have been acquired from contact with an infected mammal. To date, there have been 32 confirmed cases in dairy cows across nine U.S. states with detection of the virus in unpasteurized ("raw") milk. Additionally, cases of the virus have been also documented in goats and farm cats; see '(Waste)water surveillance for bird flu (AlH5N1)', Ad-Hoc Bulletin of the European Commission, May 2024, Vol. 2. The US Centers for Disease Control and Prevention (CDC) has confirmed one incident of zoonotic transmission, involving a young farmer who came into contact with an infected bovine, see Garg et al., 'Outbreak of Highly Pathogenic Avian Influenza A(H5N1) Viruses in U.S. Dairy Cattle and Detection of Two Human Cases - United States, 2024', CDC Morbidity and Mortality Weekly Report (MMWR), 30 May 2024, Vol, 73(21):501-505.

Methods for detecting H5N1 in milk are generally known in the art, e.g. in Guan et al., 'Cow's Milk Containing Avian Influenza A(H5N1) Virus - Heat Inactivation and Infectivity in Mice', The New England Journal of Medicine, 24 May 2024, DOI: 10.1056/NEJMc2405495; Sah et al., 'Concerns on H5N1 avian influenza given the outbreak in U.S. dairy cattle', The Lancet, 23 May 2024, Vol 35 July; p. 1-3; Hu et al., 'Highly Pathogenic Avian Influenza A (H5N1) clade 2.3.4.4b Virus detected in dairy cattle', bioRxiv preprint, 16 April 2024, doi: https://doi.org/10.1101/2024.04.16.588916; Caserta et al., 'From birds to mammals: spillover of highly pathogenic avian influenza H5N1 virus to dairy cattle led to efficient intra- and interspecies transmission', bioRxiv preprint, 22 May 2024, doi: https://doi.org/10.1101/2024.05.22.595317.

The known methods for detecting viral pathogens in dairy products are essentially based on traditional PCR or real-time PCR (RT-PCR). However, it has been shown that these methods are not optimal for the detection of viral pathogens. RT-PCR is susceptible to interference and is impaired or inhibited by various substances. This applies in particular to fats and fatty acids, which are found in significant concentrations in dairy products; see Hamza and Leifels, 'Assessment of PCR Inhibitor Removal Methods to Monitor Viruses in Environmental Water Samples: DAX-8 Outperforms Competitors', Water, Air & Soil Pollution, Vol. 235, Art. no. 20, 22 December 2023. The resulting impairment of the known detection methods prevents sensitive and quantitative detection of the virus pathogens in dairy products.

In 2024 a study has been published which shows that the H5N1 virus was detected in sewage samples from nine Texas cities. This study utilized a virome sequencing method and found that over a two-month period, the H5N1 virus was dominant over seasonal influenza viruses in a variety of wastewater samples. These findings emphasize the importance of wastewater surveillance as a method for tracking the spread of avian influenza viruses. The method is based on virome sequencing; see Tisza et al., 'Virome Sequencing Identifies H5N1 Avian Influenza in Wastewater from Nine Cities', medRxiv, preprint, 10 May 2024 (https://doi.org/10.1101/2024.05.10.24307179).

Another source reports that the US Centers for Disease Control and Prevention (CDC) has begun to expand influenza surveillance in wastewater to better track the spread of H5N1. These measures were taken particularly after outbreaks in various animal populations in order to detect potential pandemic risks at an early stage; see Run-wal, US CDC begins tracking influenza in wastewater to assess H5N1 spread, Chemical & Engineering News, ISSN 0009-2347, 21 May 2024.

Against this background, the problem underlying the invention is to provide an improved method for detecting a virus pathogen in a dairy product and water, preferably wastewater. In particular, such a method is to be provided which is less susceptible to inhibiting substances and with which a more sensitive and quantitative detection is possible.

The present invention satisfies these and other needs.

### SUMMARY OF THE INVENTION

The problem underlying the invention is solved by the use of digital polymerase chain reaction (dPCR) for detecting a virus pathogen in a dairy product and/or water.

The problem is also met by a method of detecting a virus pathogen in a dairy product or water, comprising: a) providing a sample of a dairy product, b) subjecting the sample to digital polymerase chain reaction (dPCR), said dPCR is configured to specifically amplify nucleic acid of the virus pathogen, and c) detecting the virus pathogen in the dairy product or water if an amplificate is obtained in step (b).

The properties, features, advantages and embodiments of the invention set forth in the following apply equally to the use and the method according to the invention.

Digital polymerase chain reaction (dPCR) is a biotechnological refinement of conventional polymerase chain reaction methods that can be used to directly quantify and clonally amplify nucleic acids strands including DNA, cDNA, or RNA. In contrast to RT-PCR or qPCR, dPCR uses a separation of the DNA molecules by limiting dilution and microfluidics in a large number of separate reaction vessels with a volume in the femtoliter range. In contrast to quantity determination by qPCR, this avoids variances in amplification efficiency, with lower sensitivity to PCR inhibitors but a higher detection limit. Amplification with the polymerase is carried out with the isolated nucleic acid molecules. Therefore, a digital result (amplification: yes or no) is obtained in each reaction tube, indicating the origin of the label. Statistical significance is achieved by counting a large number of reaction tubes (high-throughput screening with around 20,000 reaction tubes per square millimeter). The proportion of reaction tubes with successful amplification is proportional to the amount of nucleic acid used in the amplified sequence, which is used to determine the quantity.

The method and use according to the invention are particularly advantageous since they do not require sample purification before the pathogen nucleic acid is isolated from the sample and used as a template in the dPCR reaction. In comparison, methods of the prior art required some degree of purification of the sample in order to eliminate any PCR-inhibitory substances that are initially present in the sample, such as fats and fatty acids, respectively. The inventors showed that, surprisingly and contrary to this commonly-held belief in the art, omission of the initial purification step leads to higher nucleic acid recovery without significantly hampering downstream amplification by dPCR, thereby enabling the detection of lower pathogen concentrations in the dairy product or water. The present method is thus more sensitive than those of the prior art.

The present inventors have found that this clarification or purification step leads to a loss of genetic material, thereby preventing the detection of very low levels of nucleic acid of the virus pathogen present in the sample. In fact, comparison data show consistently higher levels of recovered pathogen nucleic acid (e.g., influenza H5N1) when the clarification or purification step is omitted. Moreover, the efficiency of the multiplex dPCR step is not affected by the absence of a clarification/purification step. Accordingly, the method according to the invention does not comprise a purification or clarification step prior to nucleic acid isolation.

As a result, the invention is hardly or significantly less susceptible to the presence of fats in the dairy product or any impurities in the water than traditional RT-PCT technology. Surprisingly, the advantage using dPCR is the higher tolerance of inhibitors in the reaction due to the partitioning in very small-volume partitions. Each partition can be seen as a nano-PCR chamber and the distribution of sample molecules and also inhibitor molecules is random following the Poisson distribution. The effects of inhibitors in the nano-PCR chamber is much less pronounced as in a bulk RT-PCR therefore outperforming RT-PCR in this invention.

Another main advantage in using dPCR for the detection of virus pathogens in dairy products or water is the absolute quantification. Indeed, it has been already shown that some human pathogens can jump from animals to humans. Surveillance systems have been established in the past few years for example for waste water monitoring. Digital PCR is making an epidemiology study much simpler as there is no need to correlate the signals for the pathogens to any kind of standard that may vary in quality and quantity from lab to lab. The absolute quantification is then very much comparable between labs, even in different countries, making the surveillance for such outbreaks much easier.

The outstanding sensitivity of dPCR is also playing a role in pathogen detection. Particularly if using high volume samples the high sensitivity of the pathogen detection is a must. dPCR enables the detection of very small amounts of viral DNA, which is particularly important for the early detection and control of viral infections.

According to the invention a "dairy product" includes food products made from or containing milk. All dairy animals are considered by the invention. The most common dairy animals are cow, water buffalo, nanny goat, and ewe. Dairy products include, without being limited thereto, common grocery store food such as milk, yogurt, cheese, and butter.

According to the invention, "water" is understood to mean any type of water, in particular waste- or sewage water, rainwater, dirty water, etc.

The method and use described herein allow for the detection of any virus pathogen present in a dairy product or water. A "pathogen" as used herein is an organism associated with, or causative of, a disease or condition of plants or animals, including humans.

"Virus pathogens" according to the invention includes all viruses potentially causing diseased when infecting plants or animals, in particular humans. Notably, they can be human or zoonotic. In particular, the invention includes RNA viruses such as Orthomyxoviridae, including influenza viruses (types A, B, C), in particular H5N1; Paramyxoviridae (measles virus, mumps virus, respiratory syncytial virus (RSV)); Coronaviridae (SARS-CoV-2, SARS-CoV, MERS-CoV); Picornaviridae (poliovirus, rhinovirus, hepatitis A virus); Flavivirida (dengue virus, west nile virus, zika virus, yellow fever virus, hepatitis C virus); Filoviridae (Ebola virus, Marburg virus); Retroviridae (human immunodeficiency virus (HIV) types 1 and 2); Rhabdoviridae (rabies virus); Togaviridae (chikungunya virus, rubella virus); Reoviridae (rotavirus). It also includes DNA viruses, such as Herpesviridae (herpes simplex virus (HSV) types 1 and 2, varicella zoster virus (VZV), Epstein-Barr virus (EBV), cytomegalovirus (CMV), human herpesvirus 6, 7, 8); Poxviridae (variola virus, vaccinia virus, molluscum contagiosum virus); Hepadnaviridae (hepatitis B Virus); Adenoviridae (adenoviruses); Papillomaviridae (human papillomavirus (HPV)); Parvoviridae (parvovirus B19); Polyomaviridae (JC virus, BK virus) and other viruses, such as Arenaviridae (lassa virus, junin virus, machupo virus), or Bunyaviridae (hantavirus, crimean-congo hemorrhagic fever virus). The dPCR in the method according to the invention is "configured to specifically amplify the nucleic acid of the viral pathogen". This means that only or essentially only the virus nucleic acid or sections thereof are amplified, but not or essentially not nucleic acid of other organisms or individuals, such as the host, e.g. the producer animal of the milk, such as the cow. "Essentially only" means that non-viral nucleic acid may be present, but only in such small quantities that the amplification of the virus nucleic acid is not materially affected. The configuration of the dPCR can be ensured by the appropriate choice of PCR primers and/or probes.

The "amplificate" refers to the specifically and selectively replicated viral nucleic acid.

In an embodiment of the invention, in a step (a') carried out after step (a) and before step (b) the dairy product and/or the water sample is pretreated with a buffer comprising a reducing agent and a proteinase, preferably proteinase K, before subjecting it to dPCR.

It is understood that in this embodiment of the invention, step (b) is carried out in a slightly modified manner, in that the sample pretreated in step (a') is subjected to dPCT.

This measure has the advantage that any interfering substances and in particular proteins present in the sample, which could possibly interfere with the dPCR, are inhibited or degraded. The invention then provides particularly reliable and accurate results. This pretreatment is of particular advantage if the sample is a dairy product or a milk sample, respectively. The pretreated sample can then be directly subjected to the dPCR, i.e. without any further step such as a nucleic acid extracting/isolating step.

The buffer used in this embodiment is commercially available, e.g. as a QlAprep&amp Buffer AB (Qiagen, cat. Nos 221513, 221515, and 221517).

In another embodiment of the invention after step (a) and, if applicable, after step (a'), and before step (b), in step (a") nucleic acid from the sample is extracted and/or isolated.

This measure has the advantage that a further optional purification step is added, which can provide a further increase in detection accuracy. This step has proven to be particularly useful when testing wastewater.

According to the invention, "nucleic acid" comprises ribonucleic acid (RNA) and, in an embodiment, deoxyribonucleic acid (DNA).

According to the invention, the "isolation" of the nucleic acid from the sample of the dairy product or water can be carried out using any methods known to a person skilled in the art.

It is understood that in this embodiment, step (b) according to the invention is carried out in a slightly modified manner, namely by subjecting the extracted and/or isolated nucleic acid to dPCR.

In an embodiment the PCR is a two-step reverse transcriptase (RT) dPCR.

Two-step reverse transcriptase (RT) digital PCR (dPCR) is a precise and sensitive method for quantifying RNA. It involves two main steps: the reverse transcription of RNA into complementary DNA (cDNA), followed by the digital PCR amplification and quantification of the cDNA. In the first step, the RNA template is reverse transcribed into cDNA using reverse transcriptase enzymes. This process can be optimized separately to ensure high efficiency and specificity in cDNA synthesis. In the second step, the cDNA generated is partitioned into thousands to millions of individual PCR reactions (in droplets, wells, or other partitioning methods). Each partition ideally contains zero or one template molecule. PCR amplification is then carried out in these partitions, and the number of partitions containing amplified product (positive reactions) is counted. The proportion of positive reactions is used to determine the absolute quantity of the target nucleic acid with high precision. In a two-step process, the reverse transcription and PCR amplification steps are optimized independently. This flexibility can result in higher efficiency and specificity in the cDNA synthesis step, improving the overall performance of the assay. This measure allows an accurate quantification of viral RNA, which is crucial for monitoring viral infections and assessing treatment efficacy. It also allows the detection and quantification of specific RNA variants, including those containing mutations or splice variants. Finally, it enables the identification and quantification of RNA biomarkers for various diseases, providing insights into disease mechanisms and potential therapeutic targets.

In an embodiment of the invention the dairy product is milk.

This measure creates a broad applicability of the method and use according to the invention. Milk is a frequently consumed product and the detection of viral pathogens in it is of great public interest. The method and use contribute to food safety. The application of the method to milk can help prevent food-borne disease and zoonotic outbreaks.

In another embodiment of the invention the milk is cow milk, preferably raw cow milk.

The measure has the advantage of allowing a targeted detection. Raw or non-pasteurized cow's milk is more susceptible to contamination due to its minimal processing, and the detection of pathogens in it is particularly important. This supports the protection of public health through the monitoring of raw milk consumed directly by the consumer.

In a further embodiment of the invention the water is wastewater.

This measure adapts the invention to a sample type that is particularly suitable for the early detection and monitoring of epidemics or pandemics. Viral material enters wastewater in various ways, especially during pandemics when infection rates are high. The most common way viruses enter wastewater is through human excretion. Infected people excrete viruses or virus fragments via stool and urine. Virus-containing droplets can get onto surfaces when sneezing, coughing or blowing their nose. When washing hands, brushing teeth or rinsing with water, these viruses can get into wastewater. Viruses can also be found in other body fluids such as blood or saliva. If these fluids are disposed of via drains or toilets, they also end up in wastewater. During a pandemic, the increased use of disinfectants and cleaning products can also lead to contaminated fluids entering wastewater.

Wastewater or, synonymously sewage water, refers to water that has been adversely affected in quality by anthropogenic influences and contains contaminants or pollutants as a result of domestic, commercial, industrial, or agricultural activities. It includes both sewage (from households, industries, or commercial establishments) and stormwater runoff, which has accumulated impurities from built or unbuilt environments. Wastewater typically contains chemical, biological, or physical impurities and generally requires treatment before it can be safely discharged into the environment.

In still another embodiment of the invention the virus pathogen is an influenza virus, preferably influenza virus subtype H5N1.

This measure focuses the specificity of the method according to the invention and its use on a virus that is responsible for particularly important human diseases. The detection of influenza, in particular the H5N1 subtype, is crucial as this subtype has a high pathogenicity and pandemic potential. This makes the invention an important preventive tool. Early detection and control of influenza viruses can help prevent outbreaks and minimize spread.

Another subject-matter of the invention is a kit for detecting a virus pathogen in a dairy product comprising primers configured to specifically amplify nucleic acid of the virus pathogen in a digital polymerase chain reaction (dPCR), and a manual comprising instructions for carrying out the method according to the invention.

The kit is user-friendly as it contains the essential components for using the method according to the invention. It also serves standardization by ensuring consistency and reproducibility of test results through standardized reagents and protocols. It facilitates access for laboratories and other facilities that need to detect viral pathogens in dairy products or water and thus increases availability.

The properties, features, advantages and embodiments of the use and the method according to the invention apply equally to the kit according to the invention.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the detailed methods and materials are now described. All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present disclosure is not entitled to antedate such publication by virtue of prior disclosure. Further, the dates of publication provided can be different from the actual publication dates, which can need to be independently confirmed.

### EMBODIMENTS

It is to be understood that this disclosure is not strictly limited to particular embodiments or examples described herein, as such can of course vary. It is also to be understood that the terminology used herein is for the purpose of describing particular embodiments only, and is not intended to be limiting, since the scope of the present invention will be limited only by the claims.

The invention is explained by means of examples resulting in additional features, characteristics and advantages of the invention. The features mentioned in the specific example are features of the invention and may be seen as general features which are not applicable in the specific example but also in an isolated manner in the context of any embodiment of the invention.

It is appreciated that certain features of the disclosure, which are, for clarity, described in the context of separate embodiments or examples, can also be provided in combination in a single embodiment. Conversely, various features of the disclosure, which are, for brevity, described in the context of a single embodiment or example, can also be provided separately or in any suitable sub-combination. All combinations of the embodiments are specifically embraced by the present disclosure and are disclosed herein just as if each and every combination was individually and explicitly disclosed. In addition, all sub-combinations are also specifically embraced by the present disclosure and are disclosed herein just as if each and every such sub-combination was individually and explicitly disclosed herein.

The invention is now further described and explained in further detail by referring to the following non-limiting examples and figures:
- Fig. 1:: dPCR instrument settings.
- Fig. 2:: Wastewater results.
Objective: Testing of CDC H5 Subtyping assays for detection of H5N1 RNA in the context of wastewater eluates and milk using QIAcuity RT-dPCR

### Testing workflow

Samples used:
*In vitro* testing: QIAcuity OneStep Advanced Probe Kit
a. H5N1 IVT
b. Wastewater eluates derived from pelleted solids collected in April 2022 from NRW wastewater samples
c. 3.8% UHT milk
d. PCR Anti-Inhibitor (Qiagen)

### Sequences of templates and assays

**Table 1: Sequences of template and assays**

| **Type** | **Oliqo name** | **Comments** |
|---|---|---|
| | | gBlock sequence. |
| | | Designed based on GenBank ID: GQ454861 sequence, corresponding to the segment 4 (HA gene) of H5N1 virus provided by Vircell supplier. |
| | | gBlock starting at position 719 of the genomic sequence. |
| | | T7 promoter added to 5' end and QNIC sequence attached to 3'end |
| **gBlock** (Positive control) | DNA639_HAgene_H5 _GQ454861 | |
| **FluAH5a Assay** | H5a_For1 | |
| | H5a_For2 | |
| | H5a_For3 | |
| | H5a_Rev1 | |
| | H5a_Rev2 | |
| | H5a_Pro1 | Based on CDC IFU, both H5 assays shall be positive. |
| | H5a_Pro2 | |
| **FluAH5b Assay** | H5a_Pro3 | Assays available in FAM and Cy 5 |
| | H5b_For1 | |
| | H5b_For2 | |
| | H5b_Rev | |
| | H5b_Pro | Human sampling control used in QIAPrep&Amp Malaria and Covid WF |

### Wastewater

### Experimental setup

Assay the sensitivity of CDC H5 Subytyping Assay to RT-dPCR inhibitors found in wastewater.

Determine if CDC H5 Subtyping Assay amplifies DNA in wastewater presumed to contain no H5 influenza strains.

Determine if Qiagen PCR Anti-inhibitor can reduce RT-dPCR inhibition of wastewater eluates on H5N1 detection.

**Table 2: Wastewater results 1**

| | **OneStep Advanced** | **Probe ONLY** | | **OneStep Advanced Probe + 1.2 µl PCR Anti-inhibitor** | | |
|---|---|---|---|---|---|---|
| | 1 | 2 | 3 | 4 | 5 | 6 |
| A | 4.44 µl Wastewater Eluate | | | 4.44 µl Wastewater Eluate | | |
| B | 2.5 µl Wastewater Eluate | | | 2.5 µl Wastewater Eluate | | |
| C | 1.25 µl Wastewater Eluate | | | 1.25 µl Wastewater Eluate | | |
| D | 0 µl Wastewater Eluate | | | 0 µl Wastewater Eluate | | |
| E | 4.44 µl Wastewater Eluate NO H5N1 RNA | | | 4.44 µl µl Wastewater Eluate NO SYNTHETIC H5N1 TEMPLATE | | |
| F | 2.5 µl Wastewater Eluate **NO SYNTHETIC H5N1 TEMPLATE** | | | 2.5 µl Wastewater Eluate **NO SYNTHETIC H5N1 TEMPLATE** | | |
| G | 1.25 µl Wastewater Eluate **NO SYNTHETIC H5N1 TEMPLATE** | | | 1.25 µl Wastewater Eluate **NO SYNTHETIC H5N1 TEMPLATE** | | |
| H | 0 µl Wastewater Eluate **NO SYNTHETIC H5N1 TEMPLATE** | | | 0 µl Wastewater Eluate **NO SYNTHETIC H5N1 TEMPLATE** | | |

### Experimental setup: assay concentrations and cycling

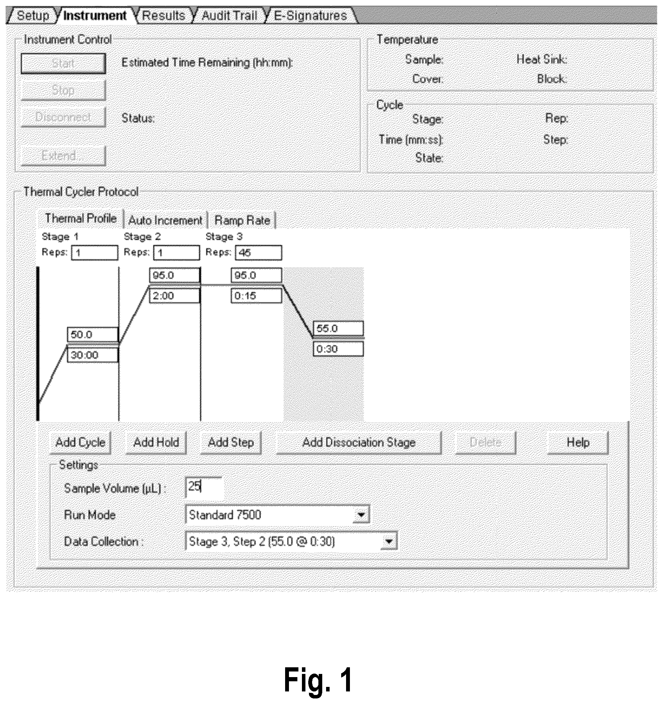

The instrument settings are depicted in Fig. 1.
a. qPCR Settingsfor ABI 7500 from CDC guide used to inform QIAcuity OneStep RT-dPCR program
b. QIAcuity program modified RT Step to from 30 min to 40 min @ 50°C.
c. Otherwise, the same program with 45 cycles and 55°C annealing extension was used

### H5A and H5B assays combined in FAM:

FluAH5a Assay (FAM)
Forward primers: 0.6 uM (x3)
Reverse primers: 0.6 uM (x2)
Probe: 0.15 uM (x3)

### FluAH5b Assay (FAM):

Forward primers: 0.6 uM (x2)
Reverse primers: 1.2 uM (x1)
Probe: 0.3 (x1)

Wastewater eluates derived from pelleted solids reduce detection of H5N1 IVT RNA by up to -70% compared to no wastewater control.

PCR Anti-inhibitor added at a 10% concentration greatly reduces RT-dPCR inhibi-tion from the wastewater.

The CDC H5 Subtyping assay does NOT amplify DNA in wastewater samples presumed to contain no H5 influenza strains
DNA target quantification is not affected by wastewater

**Table 3: Wastewater results 2**

| | | Relative Quantification | | |
|---|---|---|---|---|
| copies/µl in dPCR | OneStep Advanced Probe ONLY | OneStep Ad- vanced Probe + 10% PCR Anti-Inhibitor (1.2 µl) | OneStep Advanced Probe ONLY | OneStep Advanced Probe + 10% PCR Anti-Inhibitor (1.2 µl) |
| **GREEN CDC H5 Subtyping Assay** | | | | |
| **H5N1IVT RNA Template added to WW background** | | | | |
| 4,44µL Wastewater Eluate | 647 | 1501 | 33% | 76 % |
| 2.5µL Wastewater Eluate | 1225 | 1701 | 62 % | 86 % |
| 1.25µL Wastewater Eluate | 1492 | 1895 | 75% | 96 % |
| no Wastewater Eluate | 1699 | 1984 | 86% | 100 % |

| ****NO** H5N1 IVT RNA Template added to WW background** | | | | |
|---|---|---|---|---|
| 4.44µL Wastewater Eluate | 0.00 | 0.13 | | |
| 2.5µL Wastewater Eluate | 0.00 | 0.00 | | |
| 1.25µL Wastewater Eluate | 0.22 | 0.00 | | |
| no Wastewater Eluate | 0.00 | 0.00 | | |
| YELLOW human TERT CNV Assay | | | | |
| Positive PCR Control on DNA | | | | |

| **H5N1 IVT RNA Template added to WW background** | | | | |
|---|---|---|---|---|
| 4.44µL Wastewater Eluate | 843 | 829 | 104 % | 102 % |
| 2.5µL Wastewater Eluate | 835 | 828 | 103% | 102 % |
| 1.25µL Wastewater Eluate | 840 | 823 | 103 % | 101 % |
| no Wastewater Eluate | 830 | 837 | 102 % | 103 % |

| ****NO** H5N1 IVT RNA Template added to WW background** | | | | |
|---|---|---|---|---|
| 4.44µL Wastewater Eluate | 808 | 780 | 99% | 96 % |
| 2.5µL Wastewater Eluate | 783 | 763 | 96 % | 94 % |
| 1.25µL Wastewater Eluate | 818 | 741 | 100% | 91 % |
| no Wastewater Eluate | 833 | 815 | 102 % | 100 % |

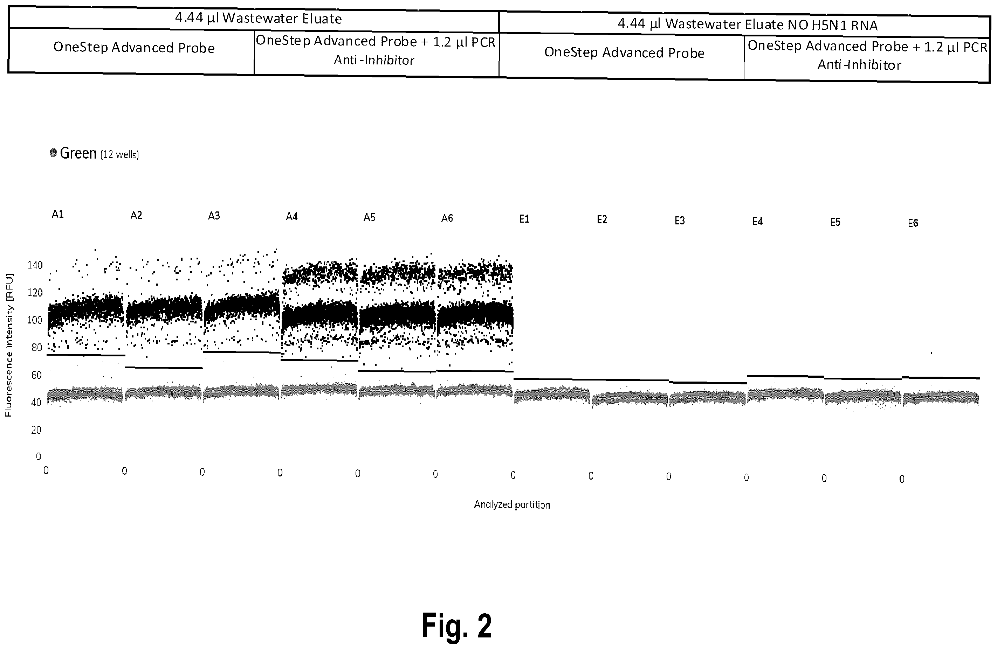

The following can be inferred from Fig. 2:
Wastewater eluates derived from pelleted solids reduce detection of H5N1 IVT RNA by up to -70% compared to no wastewater control.
PCR Anti-inhibitor added at a 10% concentration greatly reduces RT-dPCR inhibition from the wastewater.
The CDC H5 Subtyping assay does NOT amplify DNA in wastewater samples presumed to contain no H5 influenza strains

### Milk

### Experimental setup

Assay the sensitivity of CDC H5 Subytyping Assay to RT-dPCR inhibitors found in milk
Determine if using Buffer AB from QIAprep&amp can improve the ability CDC H5 Subtyping Assay to detect H5N1 RNA when milk is directly added to reaction (without extraction before)
Determine if H5N1 IVT RNA can be detected in the background of milk eluates prepped with the cador Pathogen sample prep workflow
Determine if QIAGEN PCR Anti-inhibitor can reduce RT-dPCR inhibition of milk and milk eluates on H5N1 detection

**Table 4: Milk results 1**

| | | | **OneStep Advanced Probe ONLY** | | | | **OneStep Advanced Probe + 1.2 µl PCR Anti-Inhibitor** | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 5,04 µl Milk Spike-In | | | 2,54 µl cador Pathogen Milk Eluate | | | 5,04 µl Milk Spike-In | | | 2,54 µl cador Pathogen Milk Eluate | | |
| B | 2.5 µl Milk Spike-In | | | 2.5 µl cador Pathogen Milk Eluate | | | 2.5 µl Milk Spike-In | | | 2.5 µl cador Pathogen Milk Eluate | | |
| C | 1.25 µl Milk Spike-In | | | 1.25 µl cador Pathogen Milk Eluate | | | 1.25 µl Milk Spike-In | | | 1.25 µl cador Pathogen Milk Eluate | | |
| D | 0 µl Milk Spike-In | | | 0 µl cador Pathogen Milk Eluate | | | 0 µl Milk Spike-In | | | 0 µl cador Pathogen Milk Eluate | | |
| E | 3.84 µl Milk Spike-In pre-treated with Buffer AB | | | | | | 3.84 µl Milk Spike-In pre-treated with Buffer AB | | | | | |
| F | 2.5 µl Milk Spike-In pre-treated with Buffer AB | | | | | | 2.5 µl Milk Spike-In pre-treated with Buffer AB | | | | | |
| G | 1.25 µl Milk Spike-In pre-treated with Buffer AB | | | | | | 1.25 µl Milk Spike-In pre-treated with Buffer AB | | | | | |
| H | 0 µl Milk Spike-In pre-treated with Buffer AB --> pre-treat H₂O with Buffer AB | | | | | | 0 µl Milk Spike-In pre-treated with Buffer AB-> pre-treat H2O with Buffer AB | | | | | |

Milk added directly to RT-dPCR reactions is severely inhibitors. It reduces detection of H5N1 IVT RNA by up to -98% compared to no milk control.
PCR Anti-inhibitor added at a 10% concentration DOES NOT reduces RT-dPCR inhibition of milk added directly to RT-dPCR reaction.
When milk is pretreating with Buffer AB, but not extracted, inhibition of RT-dPCR is greatly reduces which in turn greatly improves quantification of H5N1 RNA.

Eluates derived from milk with the cador Pathogen extraction kit result in the least amount of inihibition, but they are nevertheless slightly RT-dPCR inhibitory. This inhibition can be reduced by adding PCR Anti-inhibitor.

**Table 5: Milk results 2**

| copies/µl | OneStep Advanced Probe | OneStep Advanced Probe +1,2µL PCR Anti-Inhibitor | OneStep Advanced Probe | OneStep Advanced Probe +1,2µL PCR Anti-Inhibitor |
|---|---|---|---|---|
| **GREEN H5N1 RNA** | | | | |
| **Milk Spike-In** | | | | |
| 5,04µL | 103 | 116 | 5% | 6% |
| 2.5µL | 31 | 44 | 2% | 2% |
| 1.25µL | 25 | 42 | 1 % | 2% |
| 0µL | 1417 | 1941 | 73 % | 100 % |

| **Milk Spike-In pre-treated with Buffer AB** | | | | |
|---|---|---|---|---|
| 2,54µL | 1436.93 | 1406.90 | 74 % | 72 % |
| 2.5µL | 760.87 | 980.03 | 39 % | 50 % |
| 1.25µL | 997.00 | 1311.13 | 51 % | 68 % |
| 0µL | 1363 | 1438 | 70 % | 74 % |

| **cador Pathogen Milk Eluate** | | | | |
|---|---|---|---|---|
| 3,84µL | 1469 | 1671 | 76 % | 86 % |
| 2.5µL | 1396 | 1670 | 72 % | 86 % |
| 1.25µL | 1567 | 1827 | 81 % | 94 % |
| 0µL | 1753 | 1892 | 90 % | 97 % |

| **YELLOW Positive PCR Control on DNA** | | | | |
|---|---|---|---|---|
| **Milk Spike-In** | | | | |
| 5,04µL | 787 | 826 | 98 % | 103 % |
| 2.5µL | 800 | 810 | 100 % | 101 % |
| 1.25µL | 770 | 816 | 96 % | 102 % |
| 0µL | 772 | 801 | 96 % | 100 % |
| | | | | |

| **Milk Spike-In pre-treated with Buffer AB** | | | | |
|---|---|---|---|---|
| 2,54µL | 813.50 | 784.00 | 102 % | 98 % |
| 2.5µL | 822.13 | 781.63 | 103 % | 98 % |
| 1,25µL | 808.53 | 774,57 | 101 % | 97 % |
| 0µL | 816 | 762 | 102 % | 95 % |

| **cador Pathogen Milk Eluate** | | | | |
|---|---|---|---|---|
| 3,84µL | 809 | 844 | 101 % | 105 % |
| 2.5µL | 799 | 848 | 100 % | 106 % |
| 1.25µL | 788 | 839 | 98 % | 105 % |
| 0µL | 793 | 822 | 99 % | 103 % |

## Claims

1. Use of digital polymerase chain reaction (dPCR) for detecting a virus pathogen in a dairy product and/or water.

2. The use of claim 1, wherein the dairy product and/or the water is pretreated with a buffer comprising a reducing agent and a proteinase, preferably proteinase K, before subjecting it to dPCR.

3. The use of any claim 1 or 2, wherein nucleic acid is extracted and/or isolated from the dairy product and/or water before subjecting it to dPCR.

4. The use of any of claims 1 to 3, wherein said dPCR is a two-step reverse transcriptase (RT) dPCR.

5. The use of any of claims 1 to 4, wherein the dairy product is milk.

6. The use of claim 5, wherein the milk is cow milk, preferably raw cow milk.

7. The use of any of claims 1 to 6, wherein the water is wastewater.

8. The use of any of claims 1 to 7, wherein the virus pathogen is an influenza virus, preferably influenza virus subtype H5N1.

9. A method of detecting a virus pathogen in a dairy product and or water, comprising:
a) providing a sample of a dairy product or water,
b) subjecting the sample to digital polymerase chain reaction (dPCR), said dPCR is configured to specifically amplify nucleic acid of the virus pathogen, and
c) detecting the virus pathogen in the dairy product or water if an amplificate is obtained in step (b).

10. The method of claim 9, wherein after step (a) and before step (b) the following step is carried out:
(a') pretreating the sample with a buffer comprising a reducing agent and a proteinase, preferably proteinase K.

11. The method of claim 9 or 10, wherein after step (a) and, if applicable, after step (a'), and before step (b) the following step is carried out:
(a") extracting and/or isolating nucleic acid from the sample.

12. The method of any of claims 9 to 11, wherein in step (b) a two-step reverse transcriptase (RT) dPCR is carried out.

13. The method of any of claims 9 to 12, wherein the dairy product is milk, preferably cow milk, further preferably raw cow milk.

14. The method of any of claims 9 to 12, wherein the water is wastewater.

15. The method of any of any of claims 9 to 14, wherein the virus pathogen is an influenza virus, preferably influenza virus subtype H5N1.
